(19) Europäisches Patentamt / European Patent Office / Office européen des brevets

(11) **EP 1 448 223 B1**

(12) **EUROPEAN PATENT SPECIFICATION**

(45) Date of publication and mention
of the grant of the patent:
**02.01.2008 Bulletin 2008/01**

(51) Int Cl.:
*A61K 38/16* (2006.01)  *A61K 45/00* (2006.01)
*A61K 39/29* (2006.01)  *A61P 31/14* (2006.01)

(21) Application number: **02795564.0**

(22) Date of filing: **28.10.2002**

(86) International application number:
**PCT/US2002/034535**

(87) International publication number:
**WO 2003/035010 (01.05.2003 Gazette 2003/18)**

(54) **THYMOSIN AUGMENTATION OF GENETIC IMMUNIZATION**

THYMOSIN-AUGMENTATION BEI GENETISCHER IMMUNISIERUNG

AUGMENTATION DE LA THYMOSINE ASSURANT UNE IMMUNISATION GENETIQUE

(84) Designated Contracting States:
**AT BE BG CH CY CZ DE DK EE ES FI FR GB GR
IE IT LI LU MC NL PT SE SK TR**

(30) Priority: **26.10.2001 US 330638 P**

(43) Date of publication of application:
**25.08.2004 Bulletin 2004/35**

(73) Proprietor: **RHODE ISLAND HOSPITAL
Providence, RI 02903 (US)**

(72) Inventor: **WANDS, Jack, R.
Waban, MA 02168 (US)**

(74) Representative: **von Füner, Nicolai et al
v. Füner Ebbinghaus Finck Hano
Patentanwälte
Mariahilfplatz 2&3
81541 München (DE)**

(56) References cited:
**US-A- 5 849 696**

• **ENCKE J. ET AL.: 'Genetic immunization
generates cellular and humoral immune
responses against the nonstructural proteins of
the hepatitis C virus in a murine model' THE
JOURNAL OF IMMUNOLOGY vol. 161, 1998,
pages 4917 - 4923, XP002233910**

**Description**

Related applications

This application claims the benefit of provisional application 60/330,638, filed October 26, 2001.

Background

Chronic hepatitis C virus (HCV) infection is a major medical problem that leads to chronic liver disease, cirrhosis and hepatocellular carcinoma. Hepatitis C virus is the putative agent in the majority of cases of post-transfusion acquired hepatitis. Despite improvement in the quality of the blood-donor pool and the implementation of testing of donated blood, the incidence of acute infection among persons receiving transfusions is still significant. Chronic hepatitis develops in at least half the patients with acute HCV infection (representing about 90% of patients with non-A, non-B hepatitis (NANB)), and cirrhosis develops in at least 20% of this group.

A variety of drugs have been evaluated with the aim of halting or slowing the progression of HCV-related diseases. The current standard of care involves the use of interferon alpha and ribavirin. However, a significant number of individuals do not respond to such therapy. Genetic immunization has been shown to augment broad-based cellular immune responses to HCV structural and nonstructural proteins. However, the biologic activity of various HCV constructs has been weak in several experimental animal models. Tokushige, et al. (1996).

Genetic immunization is an emerging alternative to the use of traditional antigen-based vaccines such as attenuated virus or protein subunit vaccines. Genetic immunization uses naked DNA to immunize the recipient. The DNA is "naked" in the sense that it is free from any infectious delivery vehicle that can act to facilitate entry into the cell, such as viral particles. When naked DNA is administered, the recipient is able to express the plasmid DNA-encoded proteins, which can then stimulate a specific immune response composed of cytotoxic T-cells, T-helper cells, and antibodies. Use of DNA vaccines eliminates the need to purify the pathogen or immunoprotective antigen for vaccination, and there is no possibility of reversion to virulence because the DNA encodes a single viral protein.

There are numerous advantages for the use of polynucleotides for immunizations. For example, immunization can be accomplished using any antigen encoded by a polynucleotide. Furthermore, the polynucleotide encoded antigens are expressed as "pure" antigens in their native states and have undergone normal host cell modifications. Also, polynucleotides are easily and inexpensively manipulated and are stable as a dry product or in solution over a wide range of temperatures. Thus, this technology is valuable for the development of highly effective subunit vaccines.

The elicited humoral and/or cell-mediated immune response can provide protection or protective immunity against infection by pathogenic agents such as bacteria, viruses and eukaryotic organisms (e.g., parasites). The protective humoral and/or cell-mediated immune responses then interfere with the infectivity or activity of the pathogen, or limit its spread or growth, resulting in protection against subsequent challenge by the pathogen. The immune response may also combat diseases and disorders involving cells that produce specific proteins.

Genetic immunization via intramuscular injection has been shown to be effective in various animals against many viruses. For example, the immunization of guinea pigs against herpes simplex virus (HSV) type 2 infection (Boume et al. (1996)); the immunization of mice against influenza virus (Fu et al. (1997)); the vaccination of chickens against influenza viruses (Kodihalli et al. (1997)); and mammals and avians against rotaviruses (Herrmann et al. U.S. Pat. No. 5,620,896). Daheshia et al. (1997) disclose that a single application of naked DNA encoding IL-1 to the cornea of animals expressing herpetic stromal keratitis resolved the lesions affecting these animals, causing lesion remission.

A discussion of genetic immunization in general, and its use, can be found, for example, in U.S. Patent No. 5,830,876 (Weiner, et al.).

A class of polypeptide immune modifiers derived from the thymus gland, the thymosins, has been shown to trigger maturational events in lymphocytes, to augment T-cell function and to promote reconstitution of immune defects. Thymosin $\alpha_1$ (THN$\alpha_1$) is a 28 amino acid acidic polypeptide with a molecular weight of 3100 that has potent immunologic activity, including stimulation of $\alpha$- and $\gamma$-interferon production, increasing macrophage migration inhibitory factor production, inducing expression of T-cell markers, IL-2 receptors, and improving T-cell helper cell activity. The isolation, characterization and use of THN$\alpha_1$ is described, for example, in U.S. Patent No. 4,079,127.

Various antiviral agents have been used as sole therapy agents in an attempt to treat chronic HCV, including acyclovir, vidarabine, and adenine arabinoside. Sole therapy with these antiviral agents generally has been unsuccessful, either because the agent was highly toxic or resulted in some inhibition of viral replication initially, but failed to sustain viral replication inhibition long-term. See e.g. Alexander, et al., (1988).

There remains an important need for therapy for HCV that efficiently and with fewer side effects attacks the virus and modulates the immune response system and reduces the frequency of relapse.

Summary

This invention describes the use of α thymosin to augment CD4+ and CD8+ cellular immune responses to immunogenic peptides of HCV, in a preferred embodiment to the NS5 protein of HCV. It has been difficult to augment cellular immune responses to viral and cellular proteins. This invention describes the in vivo use of α thymosin to strikingly enhance cytotoxic and proliferative T-cell responses to epitopes of HCV proteins. In a preferred embodiment, the thymosin is thymosin $\alpha_1$. DNA-based vaccines are such a new technology, and so different from all previous types of vaccination (those based on proteins, peptides, or killed viruses all create humoral, or antibody-mediated, immunity while DNA-based vaccines create cell-mediated immunity) that the fact that α thymosin increases the efficacy of the vaccine provides a novel and unexpected improvement for the use of DNA-based vaccines in the treatment and prophylaxis of HCV.

Brief Description of the Figures

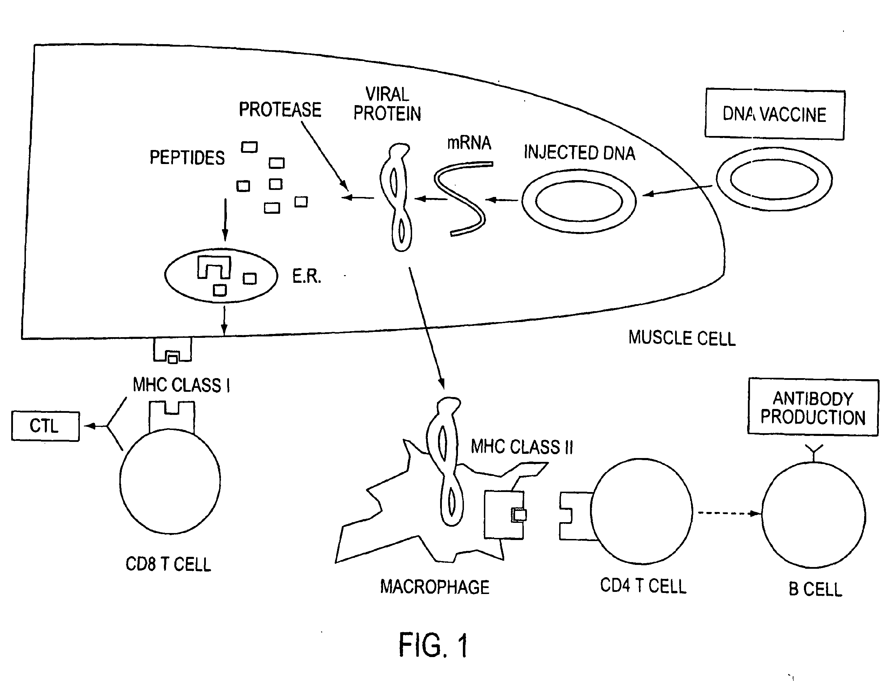

Figure 1 is an illustration of the mechanism of DNA-based immunization.

Figure 2 is a schematic representation of the hepatitis C virus (HCV) genome.

Figure 3 shows the effect of co-administration of 5 μg thymosin $\alpha_1$ i.p., with an NS5-encoding polynucleotide, on the T-cell proliferative response at three challenge concentrations (0.1 μg, 0.5 μg and 1 μg).

Figure 4 shows the effect of co-administration of 5 μg thymosin $\alpha_1$ i.p., with an NS5-encoding polynucleotide, on the cytotoxicity response at two lymphocyte effector/target (L/T) ratios.

Detailed Description

This invention describes the use of α thymosin in combination with DNA-based, or "genetic," immunization to strikingly enhance cellular immune responses to HCV. A preferred embodiment provides augmentation of DNA-based vaccines with thymosin $\alpha_1$ (thymalfasin). The invention has broad implications in prophylatic and therapeutic vaccine development, and provides a significant improvement in cellular immune responses to viral and cellular proteins following DNA-based immunization.

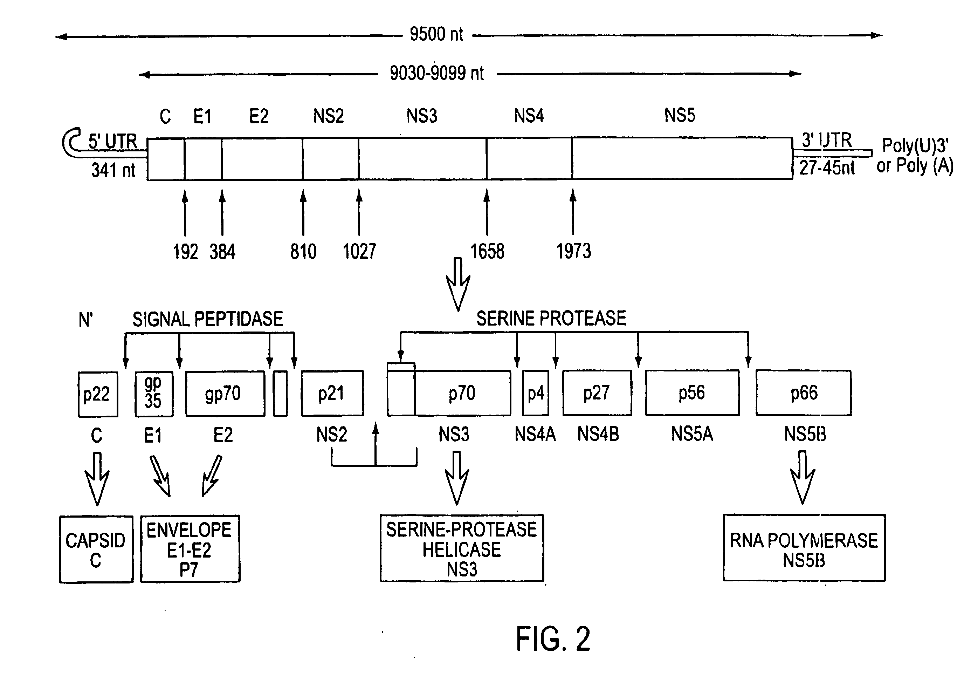

The polynucleotide that encodes an immunogenic HCV peptide, polypeptide or protein is directly administered to an animal in vivo in combination with one or more α thymosins. The polynucleotide encodes a polypeptide that shares at least one epitope with an immunogenic HCV protein to be targeted. The polynucleotide is expressed by the individual's cells to form immunogenic target proteins that elicit an immune response against HCV that is broad based. The HCV genome encodes two envelope proteins (E1 and E2) and six structural proteins (NS2, NS3, NS4A, NS4B, NS5A and NS5B). Fig. 2. Polynucleotides encoding any of these viral proteins, or combinations or fragments thereof, can be used in the present invention.

The invention is applicable to native (i.e., naturally occurring) α thymosin as well as synthetic α thymosin and recombinant α thymosin having the amino acid sequence of native α thymosins, amino acid sequences substantially similar thereto, or an abbreviated sequence from thereof, and their biologically active analogs having substituted, deleted, elongated, replaced, or otherwise modified sequences which possess bioactivity substantially similar to that of a native α thymosin. A preferred thymosin is thymosin $\alpha_1$.

The isolation, characterization and use of α thymosin is described, for example, in U.S. Patent No. 4,079,127, U.S. Patent No. 4,353,821, U.S. Patent No. 4,148,788 and U.S. Patent No. 4,116,951. The amount of α thymosin necessary to elicit the desired degree of augmentation of the effect of a DNA-based vaccine can be determined by routine dose-titration experiments. Alpha thymosin has been found to be safe for humans when administered in doses as high as 16 mg/kg body weight/day. A preferred dose of α thymosin is in the range of 0.001 mg/kg body weight/day to 10 mg/kg body weight/day, with a dose of about 0.02 mg/kg body weight/day being most preferred.

Polynucleotide sequences of the invention are DNA or RNA sequences that code for antigenic/immunogenic HCV polypeptides operably linked to transcriptional regulator sequences. These sequences may be used in association with other polynucleotide sequences coding for regulatory proteins that control the expression of these polypeptides. The regulatory protein can act by binding to genomic DNA so as to regulate its transcription; alternatively, it can act by binding to messenger RNA to increase or decrease its stability or translation efficiency.

By the term "operably linked to transcriptional and translational regulatory sequences" it is meant that a polypeptide coding sequence and minimal transcriptional and translational controlling sequences are connected in such a way as to permit polypeptide expression when the appropriate molecules (e.g., transcriptional activator proteins) are bound to the regulatory sequence(s). A polynucleotide operatively codes for a polypeptide when it has all the genetic information necessary for expression by a target cell, such as promoters and the like. The terms "promoter" or "promoter sequence" herein refers to a minimal sequence sufficient to direct transcription. A DNA polynucleotide sequence is commonly bounded by an initiation site and a termination site to form a DNA transcription unit, and is transcribed to produce a primary transcript.

The polynucleotide material delivered to the cells *in vivo* can take any number of forms, and the present invention is not limited to any particular polynucleotide coding for any particular HCV polypeptide, though polynucleotides encoding the NS5 protein, or a fragment thereof, are preferred. Plasmids containing genes coding for HCV antigens or immunogens have been reported in the literature and can be readily obtained by those of skill in the art (see, e.g., Tokushige, et al., 1996).

The polynucleotide can encode one or multiple antigens, such as antigens from two or more different viral proteins. Alternatively, the polynucleotide may contain two or more different DNA sequences, one sequence encoding an antigen and the other(s) encoding polypeptides which may or may not be antigenic. For example, the vector may encode two (or more) HCV antigens. In another embodiment, the other polypeptide(s) may serve to enhance an immune response against HCV (e.g., helper epitopes, cytokines, carrier polypeptides, cholera toxin subunits, or other immunostimulants).

The polynucleotide can additionally be inserted into a vector that includes sequences for expression of the polynucleotide. When two or more polypeptide-encoding DNA sequences are present in one vector, the transcription of each antigen-encoding DNA sequence may be directed from its own promoter for expression of two or more non-fused polypeptides. Alternatively, one promoter may drive the expression of two or more antigen-encoding DNA sequences joined in frame to each other to express a fusion protein.

The polynucleotides used in these methods can be sequences which do not integrate into the genome of the host cell. These may be non-replicating DNA sequences, or specific replicating sequences genetically engineered to lack the genome-integration ability. The polynucleotide can be administered to the subject in the presence of adjuvants or other substances that have the capability of promoting nucleic acid uptake or recruiting immune system cells to the site of the inoculation. It should be understood that the polynucleotide itself is expressed in the host cell by transcription factors provided by the host cell, or provided by a DNA transcription unit.

According to the invention, both expressible DNA and mRNA can be delivered to cells to form a polypeptide translation product therein. If the nucleic acids contain the proper control sequences, they will direct the synthesis of relatively large amounts of the encoded protein.

The dosage of the immunogenic polypeptide can be readily determined by a clinician or veterinarian employing animal models or other test systems that are well known to the art. Formulations will contain an effective amount of the DNA in an aqueous solution. The amount of DNA to be administered depends upon factors such as the age, weight and physical condition of the subject considered for vaccination. The amount of DNA also depends upon the capacity of the subject's immune system to synthesize antibodies, and the degree of protection desired. Effective dosages can be readily established by one of ordinary skill in the art through routine trials establishing dose response curves. A preferred dose range is between 1 $\mu$g/kg subject body weight and 1 mg/kg subject body weight. The subject may be immunized by administration of the DNA at one time, or by multiple administrations. Multiple administrations may be required to maintain a state of immunity by the subject to a particular pathogen.

The compositions of and method for constructing heterologous polynucleotides for successful transformations is well know to those skilled in the art, and the same compositions and methods of construction may be used to produce the polynucleotides useful herein. The specific composition of the polynucleotide is not central to the present invention and the invention is not dependent upon the composition of the specific transforming polynucleotide used. Suitable components of the polynucleotide including promoters, polyadenylation sequences, termination signals, splicing signals, selectable markers, reporter genes, enhancers, viral replicons, introns, and bacterial plasmid sequences are well known in the art. Sambrook et al. (1989) provides suitable methods of heterologous polynucleotide construction.

Polynucleotides can be produced by a number of known methods. For example, DNA encoding a preselected antigen can be inserted into an expression vector (see, for example, Sambrook et al. (1989)). With the availability of automated nucleic acid synthesis equipment, DNA can be synthesized directly when the nucleotide sequence is known, or by a combination of PCR, cloning, and fermentation. Moreover, when the sequence of the preselected polypeptide is known, a suitable coding sequence for the polynucleotide can be inferred.

When the polynucleotide is mRNA, it can be readily prepared from the corresponding DNA in vitro. For example, conventional techniques use phage RNA polymerases SP6, T3, or T7 to prepare mRNA from DNA templates in the presence of the individual ribonucleoside triphosphates. An appropriate phage promoter, such as a T7 origin of replication site is placed in the template DNA immediately upstream of the gene to be transcribed. Systems using T7 in this manner are well known, and are described in the literature.

A further aspect of the invention relates to an immunological composition which, when introduced into an individual capable or having induced within it an immunological response, induces an immunological response in such individual to an HCV protein coded therefrom, wherein the composition comprises a polynucleotide which codes for and expresses one or more antigens/immunogens of HCV, in combination with one or more $\alpha$ thymosins. The immunological response may be used therapeutically or prophylactically and may take the form of antibody immunity or cellular immunity such as that arising from CTL or CD4+ T-cells.

Example 1

**Plasmid Construction**

As a source of viral genes, a plasmid designated pBRTM/HCV1-3011 covering the full-length open reading frame (ORF) of HCV was used to clone into expression vectors (Grakoui, et al., 1993). Construct pAp031-Ns5 was PCR cloned after inserting engineered start and stop codons as well as restriction enzyme sites using the following primers: for NS5, 5'-T CAG TCT AGA ATG TCC GGC TCC TGG CTA AGG GA-3' (XbaI) [SEQ ID No. 1] and 5'-A GCT ACG CGT TCA CCG GTT GGG GAG GAG GT-3' (MluI) [SEQ ID No. 2]. After PCR amplification using a high-fidelity PCR system (Bochringer Mannheim, Indianapolis, IN), the cDNA fragments were inserted into the plasmid expression vector pAp031 containing a Rous sarcoma virus enhancer element and a CMV promoter. Constructs were transformed into DH5α cells, and plasmid DNA was subsequently purified by either 2x cesium chloride centrifugation or with a Qiagen Giga kit using the Endofree buffer system (Santa Clara, CA). Correct insertion of cDNAs coding for nonstructural proteins was verified by sequencing analysis using standard methods. To establish stable NS5 expressing cell lines as target cells for the CTL assays, the nonstructural protein-encoding gene fragments were also cloned into the pcDNA3 and pcDNA3.1/Zeo(-) expression vectors (Invitrogen, San Diego, CA) with a neomycin selectable marker. An *Xba*I and *Mlu*I fragment NS5 was subcloned into the *Nhe*I/*Mlu*I site of Litmus-38 vector (New England Biolabs, Beverly, MA), cut with *Eco*R1 and *Sal*I, and ligated into the *Eco*RI/*Xho*I multiple cloning site of pcDNA3 and pcDNA3, I/Zeo(-), respectively. An *Hba*I and *Bam*HI fragment containing NS4 was ligated into Litmus-29 (New England Biolabs), recut with *Kpn*I and EcoRI, and subsequently ligated into the pc DNA 3 vector. Plasmids were designated pcDNA3.1/Zeo(-)-NS5. DNA expression plasmid encoding for murine IL-2 (pcD/3-I12) was cloned and purified as previously described (Geissler, et al., 1997a, 1997b, 1997c).

**Genetic Immunization**

To enhance the cellular uptake of plasmid DNA, the quadriceps muscle of Balb/c mice was injected at multiple sites with a total of 100 μL of 0.25% hupivacaine. Four days later, the plasmid constructs were injected into the same region at five different sites in a final volume of 100 μL 0.9% NaCl. Two weeks later the mice were boosted in the opposite leg with plasmid DNA. The mice were killed ten days after the last immunization. There were three groups of mice each containing five animals. Group 1 = mice immunized with 100 μg mock DNA; Group 2 = mice immunized with 50 μg pAp031NS5 and 50 μg mock DNA; Group 3 = mice immunized with 50 μg pAp031 NS5 and 50 μg mock DNA, and received 5 μg thymosin $\alpha_1$ i.p. twice/week. Animals were immunized three times two weeks apart, and studies were performed ten days after the final immunization.

**T-Cell Proliferation Assay**

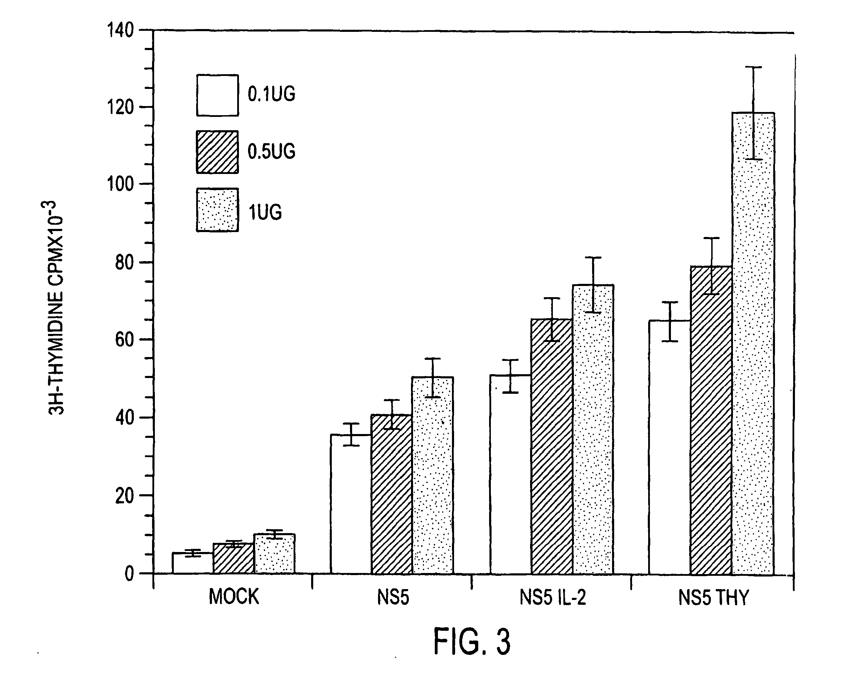

Mice were anesthetized with isoflurane (Aerrane, Anaquest, NJ), blood was removed by retrobulbar puncture and spleen cells were harvested. Red blood cells were removed by incubation in 8.3% $NH_4Cl$/0.17 mol/L Tris (pH 7.4) for 10 minutes at 37 °C. Spleen cells were cultured in triplicate using 96-well flat bottom plates at $5 \times 10^5$ cells per well in 100 μL complete (Dulbeccos Modified Eagle's Medium, Mediatech, Washington, DC) containing 10% FCS. Spleen cells were stimulated with recombinant NS5 at different concentrations (1, 5, 10 μg/mL). Finally, 2-mercaptoethanol was added to a final concentration of 50 μmol/L. As a control for antigen specificity, effector cells were stimulated with 10 μg/mL of recombinant β-subunit of human chorionic gonadotropin which is secreted from the cell and has been shown to be a strong T-cell immunogen (Geissler, et al., 1997d). Spleen cells were stimulated for 3 days. After adding [3H]-thymidine (1 μCi/well) cells were incubated for 18 hours. The [3H]-thymidine incorporation into DNA was measured after harvesting. Fig. 3. Incorporation of radioactivity was corrected for background activity (Δ cpm).

**Cytotoxicity Assay**

Spleen cells from immunized mice were suspended in complete DMEM containing 10% FCS and 50 μmol/L 2-mercaptoethanol; the cells were then analyzed for cytotoxic activity 5 days after in vitro stimulation. Recombinant murine IL-2 was added once at a concentration of 5 U/mL and responder cells ($4 \times 10^7$) were co-cultured with $6.25 \times 10^6$ syngeneic cells stably expressing NS5 after treatment with 20,000 rad. Cytotoxic effector lymphocyte populations were harvested after 5 days of incubation. A 4-hour $^{51}$Cr-release assay was performed in a 96-well round bottom plate using as a target cell line $^{51}$Cr-labeled SP2-NS5 cells. CTL assays were performed at lymphocyte effector:target (L/T) ratios of 1:10 and 1:100. Percent cytotoxicity was calculated as:

$$\frac{\text{Experimental release - spontaneous release}}{\text{Maximum release - spontaneous release}}$$

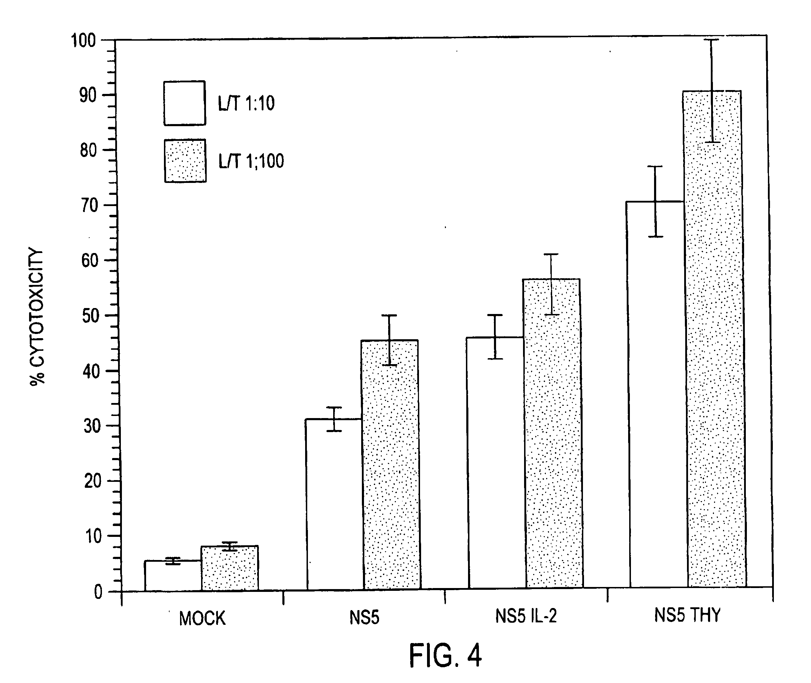

Experimental release represents the mean counts per minute released by target cells in presence of effector cells. Total release represents the radioactivity released after total lysis of target cells with 5% TritonX-100. Fig. 4.

Spontaneous release represents the radioactivity present in medium derived from target cells only.

**Statistical Analysis**

To compare the results between the different groups, we used a nonparametric Mann-Whitney U test. $P < .05$ was considered statistically significant. Numbers for P values according to CD4+ and CD8+ T-cell responses are derived from all recombinant NS5 concentrations used for stimulation and E:T ratios respectively.

The results shown in Figures 3 and 4 demonstrate the dramatic improvement in immune response that can be achieved by co-administration of $\alpha$ thymosin in a DNA-based immunization treatment. Co-administration of $\alpha$ thymosin increased the CD4 proliferative response by approximately 100% against all three challenge concentrations. Fig. 3. The percent cytotoxicity was also increased by 100%, or more, by co-administration of $\alpha$ thymosin. Fig. 4.

References

Alexander, G.J.M., et al. (1988), American J. Med. 85-2A:143-146.

Boume, N., L.R. Stanberry, D.I. Bernstein, D. Lew (1996), DNA immunization against experimental genital herpes simplex virus infection, J. Infect. Dis. 173:800-807.

Daheshia, M., N. Kuklin, S. Kanangat, E. Manickan, B.T. Rouse (1997), Suppression of ongoing ocular inflammatory disease by topical administration of plasmid DNA encoding IL-10, J. Immunol. 159:1945-1952.

Fu, T.M., A. Friedman, J.B. Ulmer, M.A. Liu, J.J. Donnely (1997), Protective cellular immunity: cytotoxic T-lymphocyte responses against dominant and recessive epitopes of influenza virus nucleoprotein induced by DNA immunization, J. Virol. 71:2715-2721.

Grakoui A, Wychowski C, Lin C, Feinstone SM, Rice CM (1993) Expression and identification of hepatitis C virus polyprotein cleavage products. J. Virol. 67:1385.

Geissler M, Tokushige K, Wands JR. Cellular and humoral immune response to hepatitis B virus structural proteins in mice following DNA-based immunization (1997a), Gastroenterology 112:1307-20.

Geissler M, Gesien A, Tokushige K, Wands JR (1997b), Enhancement of cellular and humoral immune responses to hepatitis C virus (HCV) core protein using DNA-based vaccines augmented with cytokine expressing plasmids. J. Immunol. 258:1231-7.

Geissler M, Cesion A, Wands JR (1997c), Chronic ethanol effects on cellular immune responses to hepatitis B virus envelope protein; an immunologic mechanism for induction of persistent viral infection in alcoholics. Hepatology 26: 764-70.

Geissler M, Wands G, Gesien A, de la Monte S, Bellet D, Wands JR (1997d), Genetic immunization with the free human chorionic gonadotropin $\beta$-subunit elicits cytotoxic T lymphocyte responses and protects against tumor formation in mice. Lab. Invest. 76:859-71.

Kodihalli, S., J.R. Hayes, H.L. Robinson, R.G. Webster (1997), Cross-protection among lethal H5N2 influenza viruses induced by DNA vaccine to the hemagglutinin, J. Virol. 71:3391-3396.

Sambrook, et al., Molecular cloning, a laboratory manual, 2d, Cold Spring Harbor Press (1989).

Tokushige, et al. (1996), Expression and immune response to hepatitis C virus core DNA-based vaccine, Hepatology 24:14-20.

United States Patent No. 4,079,127 (Goldstein, et al.)

United States Patent No. 4,116,951 (Wang)

United States Patent No. 4,148,788 (Wang)

United States Patent No. 4,353,821 (Birr, et al.)

United States Patent No. 5,620,896 (Herrmann, et al.)

United States Patent No. 5,830,876 (Weiner, et al.)

**Claims**

1. Use of a polynucleotide encoding one or more hepatitis C virus peptides, along with one or more α thymosins, in the preparation of a pharmaceutical combination for immunizing a subject susceptible to hepatitis C virus infection against such infection.

2. The use of claim 1, wherein the polynucleotide encodes one or more hepatitis C virus envelope proteins.

3. The use of claim 1, wherein the polynucleotide encodes one or more hepatitis C virus proteins selected from the group consisting of the NS3 protein, the NS4A protein, the NS4B protein, the NS5A protein and the NS5B protein.

4. The use of claim 3, wherein the polynucleotide encodes one or more NS5 proteins.

5. The use of any of claims 1-4, wherein the α thymosin is thymosin α1.

6. The use of any of claims 1-4, wherein the thymosin is at a dose of 0.001 mg/kg body weight/day and 10 mg/kg body weight/day.

7. The use of claim 6, wherein the thymosin is at a dose of about 0.02 mg/kg body weight/day.

8. A pharmaceutical combination for immunizing a subject susceptible to hepatitis C virus infection against such infection comprising a polynucleotide encoding one or more hepatitis C virus peptides, along with one or more α thymosins.

9. The combination of claim 8, wherein the polynucleotide encodes one or more hepatitis C virus envelope proteins.

10. The combination of claim 8, wherein the polynucleotide encodes one or more hepatitis C virus proteins selected from the group consisting of the NS3 protein, the NS4A protein, the NS4B protein, the NS5A protein and the NS5B protein.

11. The combination of claim 10, wherein the polynucleotide encodes one or more NS5 proteins.

12. The combination of any of claims 8-11, wherein the a thymosin is thymosin α1.

13. The combination of any of claims 8-11, wherein the thymosin is at a dose of 0.001 mg/kg body weight/day and 10 mg/kg body weight/day.

14. The combination of claim 13, wherein the thymosin is at a dose of about 0.02 mg/kg body weight/day.

**Patentansprüche**

1. Verwendung eines ein oder mehrere Hepatitis C-Virus-Peptide codierenden Polynucleotids zusammen mit einem oder mehreren α-Thymosinen für die Herstellung eines pharmazeutischen Gemisches für die Immunisierung eines für Hepatitis C-Virusinfektion anfälligen Patienten gegen eine solche Infektion.

2. Verwendung nach Anspruch 1, bei dem das Polynucleotid ein oder mehrere Hepatitis C-Virus-Enveloproteine codiert.

3. Verwendung nach Anspruch 1, bei dem das Polynucleotid ein oder mehrere Hepatitis C-Virus-Proteine codiert, ausgewählt aus der Gruppe NS3-Protein, NS4A-Protein, NS4B-Protein, NS5A-Protein und NS5B-Protein.

4. Verwendung nach Anspruch 3, bei dem das Polynucleotid ein oder mehrere NS5-Proteine codiert.

5. Verwendung nach einem der Ansprüche 1-4, bei der das α-Thymosin Thymosin α1 ist.

6. Verwendung nach einem der Ansprüche 1-4, bei der das Thymosin in einer Dosis von 0,001 mg/kg Körpergewicht/Tag und 10 mg/kg Körpergewicht/Tag vorliegt.

7. Verwendung nach Anspruch 6, bei der das Thymosin in einer Dosis von ca. 0,02 mg/kg Körpergewicht/Tag vorliegt.

8. Pharmazeutisches Gemisch zur Immunisierung eines für Hepatitis C-Virus-Infektion anfälligen Patienten, das ein Polynucleotid, das ein oder mehrere Hepatitis C-Virus-Peptide codiert, zusammen mit einem oder mehreren α-Thymosinen umfaßt.

9. Gemisch nach Anspruch 8, worin das Polynucleotid ein oder mehrere Hepatitis C-Virus-Enveloproteine codiert.

10. Gemisch nach Anspruch 8, worin das Polynucleotid ein oder mehrere Hepatitis C-Virus-Proteine codiert, ausgewählt aus der Gruppe NS3-Protein, NS4A-Protein, NS4B-Protein, NS5A-Protein und NSSB-Protein.

11. Gemisch nach Anspruch 10, worin das Polynucleotid ein oder mehrere NS5-Proteine codiert.

12. Gemisch nach einem der Ansprüche 8 - 11, worin das α-Thymosin Thymosin α1 ist.

13. Gemisch nach einem der Ansprüche 8 - 11, worin das Thymosin in einer Dosis von 0,001 mg/kg Körpergewicht/Tag und 10 mg/kg Körpergewicht/Tag vorliegt.

14. Gemisch nach Anspruch 13, worin das Thymosin in einer Dosis von ca. 0,02 mg/kg Körpergewicht/Tag vorliegt.


**Revendications**

1. Utilisation d'un polynucléotide codant pour un ou plusieurs peptides du virus de l'hépatite C, avec une ou plusieurs thymosines α, dans la préparation d'une combinaison pharmaceutique destinée à immuniser un sujet sensible à une infection par le virus de l'hépatite C contre une telle infection.

2. Utilisation selon la revendication 1, où le polynucléotide code pour une ou plusieurs protéines de l'enveloppe du virus de l'hépatite C.

3. Utilisation selon la revendication 1, où le polynucléotide code pour une ou plusieurs protéines du virus de l'hépatite C sélectionnées dans le groupe constitué par la protéine NS3, la protéine NS4A, la protéine NS4B, la protéine NS5A et la protéine NS5B.

4. Utilisation selon la revendication 3, où le polynucléotide code pour une ou plusieurs protéines NS5.

5. Utilisation selon l'une quelconque des revendications 1-4, où la thymosine α est la thymosine α1.

6. Utilisation selon l'une quelconque des revendications 1-4, où la thymosine se trouve à une dose de 0,001 mg/kg de poids corporel/jour à 10 mg/kg de poids corporel/jour.

7. Utilisation selon la revendication 6, où la thymosine se trouve à une dose d'environ 0,02 mg/kg de poids corporel/jour.

8. Combinaison pharmaceutique destinée à immuniser un sujet sensible à une infection par le virus de l'hépatite C contre une telle infection, comprenant un polynucléotide codant pour un ou plusieurs peptides du virus de l'hépatite C, avec une ou plusieurs thymosines α.

9. Combinaison selon la revendication 8, où le polynucléotide code pour une ou plusieurs protéines de l'enveloppe du virus de l'hépatite C.

10. Combinaison selon la revendication 8, où le polynucléotide code pour une ou plusieurs protéines du virus de l'hépatite C sélectionnées dans le groupe constitué par la protéine NS3, la protéine NS4A, la protéine NS4B, la protéine NS5A et la protéine NS5B.

11. Combinaison selon la revendication 10, où le polynucléotide code pour une ou plusieurs protéines NS5.

12. Combinaison selon l'une quelconque des revendications 8-11, où la thymosine α est la thymosine α1.

13. Combinaison selon l'une quelconque des revendications 8-11, où la thymosine se trouve à une dose de 0,001 mg/kg de poids corporel/jour à 10 mg/kg de poids corporel/jour.

14. Combinaison selon la revendication 13, où la thymosine se trouve à une dose d'environ 0,02 mg/kg de poids corporel/jour.

FIG. 1

EP 1 448 223 B1

9500 nt

9030-9099 nt

C  E1  E2  NS2  NS3  NS4  NS5

5' UTR  3' UTR  Poly(U)3'
341 nt  27-45nt  or Poly (A)

192 384  810 1027  1658  1973

N'  SIGNAL PEPTIDASE  SERINE PROTEASE

| p22 | gp35 | gp70 | | p21 | p70 | p4 | p27 | p56 | p66 |

C  E1  E2  NS2  NS3  NS4A  NS4B  NS5A  NS5B

CAPSID C

ENVELOPE E1-E2 P7

SERINE-PROTEASE HELICASE NS3

RNA POLYMERASE NS5B

FIG. 2

FIG. 3

FIG. 4

EP 1 448 223 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 60330638 A
- US 5620896 A
- US 5830876 A
- US 4079127 A
- US 4353821 A
- US 4148788 A
- US 4116951 A

### Non-patent literature cited in the description

- **ALEXANDER, G.J.M. et al.** *American J. Med.,* 1988, vol. 85-2A, 143-146
- **BOUME, N. ; L.R. STANBERRY ; D.I. BERNSTEIN ; D. LEW.** DNA immunization against experimental genital herpes simplex virus infection. *J. Infect. Dis.,* 1996, vol. 173, 800-807
- **DAHESHIA, M. ; N. KUKLIN ; S. KANANGAT ; E. MANICKAN ; B.T. ROUSE.** Suppression of ongoing ocular inflammatory disease by topical administration of plasmid DNA encoding IL-10. *J. Immunol.,* 1997, vol. 159, 1945-1952
- **FU, T.M. ; A. FRIEDMAN ; J.B. ULMER ; M.A. LIU ; J.J. DONNELY.** Protective cellular immunity: cytotoxic T-lymphocyte responses against dominant and recessive epitopes of influenza virus nucleoprotein induced by DNA immunization. *J. Virol.,* 1997, vol. 71, 2715-2721
- **GRAKOUI A ; WYCHOWSKI C ; LIN C ; FEINSTONE SM ; RICE CM.** Expression and identification of hepatitis C virus polyprotein cleavage products. *J. Virol.,* 1993, vol. 67, 1385
- **GEISSLER M ; TOKUSHIGE K ; WANDS JR.** Cellular and humoral immune response to hepatitis B virus structural proteins in mice following DNA-based immunization. *Gastroenterology,* 1997, vol. 112, 1307-20
- **GEISSLER M ; GESIEN A ; TOKUSHIGE K ; WANDS JR.** Enhancement of cellular and humoral immune responses to hepatitis C virus (HCV) core protein using DNA-based vaccines augmented with cytokine expressing plasmids. *J. Immunol.,* 1997, vol. 258, 1231-7
- **GEISSLER M ; CESION A ; WANDS JR.** Chronic ethanol effects on cellular immune responses to hepatitis B virus envelope protein; an immunologic mechanism for induction of persistent viral infection in alcoholics. *Hepatology,* 1997, vol. 26, 764-70
- **GEISSLER M ; WANDS G ; GESIEN A ; DE LA MONTE S ; BELLET D ; WANDS JR.** Genetic immunization with the free human chorionic gonadotropin β-subunit elicits cytotoxic T lymphocyte responses and protects against tumor formation in mice. *Lab. Invest.,* 1997, vol. 76, 859-71
- **KODIHALLI, S. ; J.R. HAYES ; H.L. ROBINSON ; R.G. WEBSTER.** Cross-protection among lethal H5N2 influenza viruses induced by DNA vaccine to the hemagglutinin. *J. Virol.,* 1997, vol. 71, 3391-3396
- **SAMBROOK et al.** Molecular cloning, a laboratory manual. Cold Spring Harbor Press, 1989
- **TOKUSHIGE et al.** Expression and immune response to hepatitis C virus core DNA-based vaccine. *Hepatology,* 1996, vol. 24, 14-20